# EUROPÄISCHE PATENTSCHRIFT

(11) **EP 0 219 782 B1**
(45) Veröffentlichungstag und Bekanntmachung des Hinweises auf die Patenterteilung: **29.09.1993**
(21) Anmeldenummer: 86114097.8
(22) Anmeldetag: 11.10.1986
(51) Int. Cl.: A61K 37/64

(54) **Verwendung von Angiotensin-Converting-Enzym-Inhibitoren zur Behandlung von Atherosklerose, Thrombose und der peripheren Gefässkrankheit**
Use of angiotensin-converting enzyme inhibitors in the treatment of atherosclerosis, thrombosis and peripheral vascular disease
Utilisation des inhibiteurs de l'enzyme convertissant l'angiotensine pour le traitement de l'athérosclérose, de la thrombose et de la maladie vasculaire-périphérique

(30) Priorität: 15.10.1985 DE 3536687
(43) Veröffentlichungstag der Anmeldung: 29.04.1987
(73) Patentinhaber: HOECHST AKTIENGESELLSCHAFT, 65926 Frankfurt am Main (DE)
(72) Erfinder: Schölkens, Bernward, Dr., D-6233 Kelkheim (Taunus) (DE)

(56) Entgegenhaltungen:
- JOURNAL OF CARDIOVASCULAR PHARMACOLOGY, Band 6, Nr. 5, 1984, Seiten 840-843, Raven Press, New York, US; N. SOMEYA et al.: "Suppressive effect of captopril on platelet aggregation in essential hypertension"
- ANGIOLOGY, Band 36, Nr. 5, Mai 1985, Seiten 293-296, New York, US; M. CATALANO et al.: "Captopril for the treatment of patients with hypertension and peripheral vascular disease"
- DTSCH. MED. WOCHENSCHR., Band 109, Nr. 22, 1984, Seiten 857-860, Georg Thieme Verlag, Stuttgart, DE; G. THUBESTEIN et al.: "Behandlung des Raynaud-Syndroms mit Captoril"
- BRITISH MEDICAL JOURNAL, Band 284, Nr. 6312, 30. Januar 1982, Seiten 310-311, London, GB, S. MIYAZAKI et al.: "Relief from digital vasospasm by treatment with captopril and its complete inhibition by serine proteinase inhibitores in Reynaud's phenomenon"
- FOLIA OPHTHALMOL. JPN., Band 34, Nr. 2, 1983, Seiten 290-297, Osaka, JP, K. YAMAGAMI et al.: "Further study on antihypertensive and antisclerotic effects of captopril on the retinal arteriole in SHRSP"
- ATHEROSCLEROSIS, Band 40, 1981, Seiten 263-271, Elsevier/North-Holland Scientific Publishers, Ltd., Amsterdam, NL. S. WADA et al.: "The lipid-lowering profile in rodents"
- Hochdruck 6: 27 (1985), Schölkens, B.A. et al.

## Beschreibung

Die Erfindung betrifft ein Verfahren zur Behandlung der Atherosklerose, der Thrombose und/oder der peripheren Gefäßkrankheit durch perorale oder parenterale Anwendung von Verbindungen, die das Angiotensin-Converting-Enzymne hemmen. Dabei kommen besonders Verbindung der Formel I in Betracht, 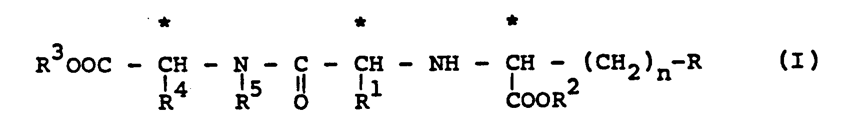
in welcher
- n =: 1 oder 2 ist,
- R =: Aryl mit 6 - 12 C-Atomen, das durch (C₁-C₄)-Alkyl, (C₁-C₄)-Alkoxy, Hydroxy, Halogen, Nitro, Amino, Aminomethyl, (C₁-C₄)-Alkylamino, Di-(C₁-C₄)-alkylamino, (C₁-C₄)-Alkanoylamino, Methylendioxy, Carboxy, Cyano und/oder Sulfamoyl mono-, di- oder trisubstituiert sein kann,
- R¹: Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
(C₃-C₉)-Cycloalkyl-(C₁-C₄)-alkyl,
(C₅-C₉)-Cycloalkenyl-(C₁-C₄)-alkyl,
gegebenenfalls teilhydriertes Aryl mit 6 - 12 C-Atomen, das wie oben bei R beschrieben substituiert sein kann,
(C₆-C₁₂)-Aryl-(C₁-C₄)-alkyl oder (C₇-C₁₃)-Aroyl-(C₁ oder C₂)alkyl die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, die gegebenenfalls geschützte Seitenkette natürlich vorkommenden α-Aminosäure R¹-CH(NH₂)-COOH bedeuten,
- R² und R³: gleich oder verschieden sind und Wasserstoff,
Alkyl mit 1 - 6 C-Atomen,
Alkenyl mit 2 - 6 C-Atomen,
Di-(C₁-C₄)-alkylamino-(C₁-C₄)-alkyl,
(C₁-C₅)-Alkanoyloxy-(C₁-C₄)-alkyl,
(C₁-C₆)-Alkoxy-carbonyloxy-(C₁-C₄)-alkyl,
(C₇-C₁₃)-Aroyloxy-(C₁-C₄)-alkyl,
(C₆-C₁₂)-Aryloxycarbonyloxy-(C₁-C₄)-alkyl,
Aryl mit 6 - 12 C-Atomen,
(C₆-C₁₂)-Aryl-(C₁-C₄)-alkyl,
(C₃-C₉)-Cycloalkyl oder
(C₃-C₉)-Cycloalkyl-(C₁-C₄)-alkyl
bedeuten und
worin R⁴ und R⁵ zusammen mit den sie tragenden Atomen für ein System aus der Reihe Tetrahydroisochinolin, Decahydroisochinolin, Octahydroindol, Octahydrocyclopenta[b]pyrrol, 2-Aza-spiro[4.5]decan, 2-Azaspiro[4.4]nonan, Spiro[(bicyclo-[2.2.1]heptan)-2,3'-pyrrolidin], Spiro[(bicyclo-[2.2.2]octan)-2,3'pyrrolidin], 2-Azatricyclo[4,3,0,1^{6.9}]decan, Decahydrocyclopenta[b]pyrrol, Octahydroisoindol, Octahydrocyclopenta[c]pyrrol, 2,3,3a,4,5,7a-Hexahydroindol und 2-Azabicyclo[3.1.0]hexan stehen.

Bei den Verbindungen, die mehrere chirale Atome besitzen, kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf.

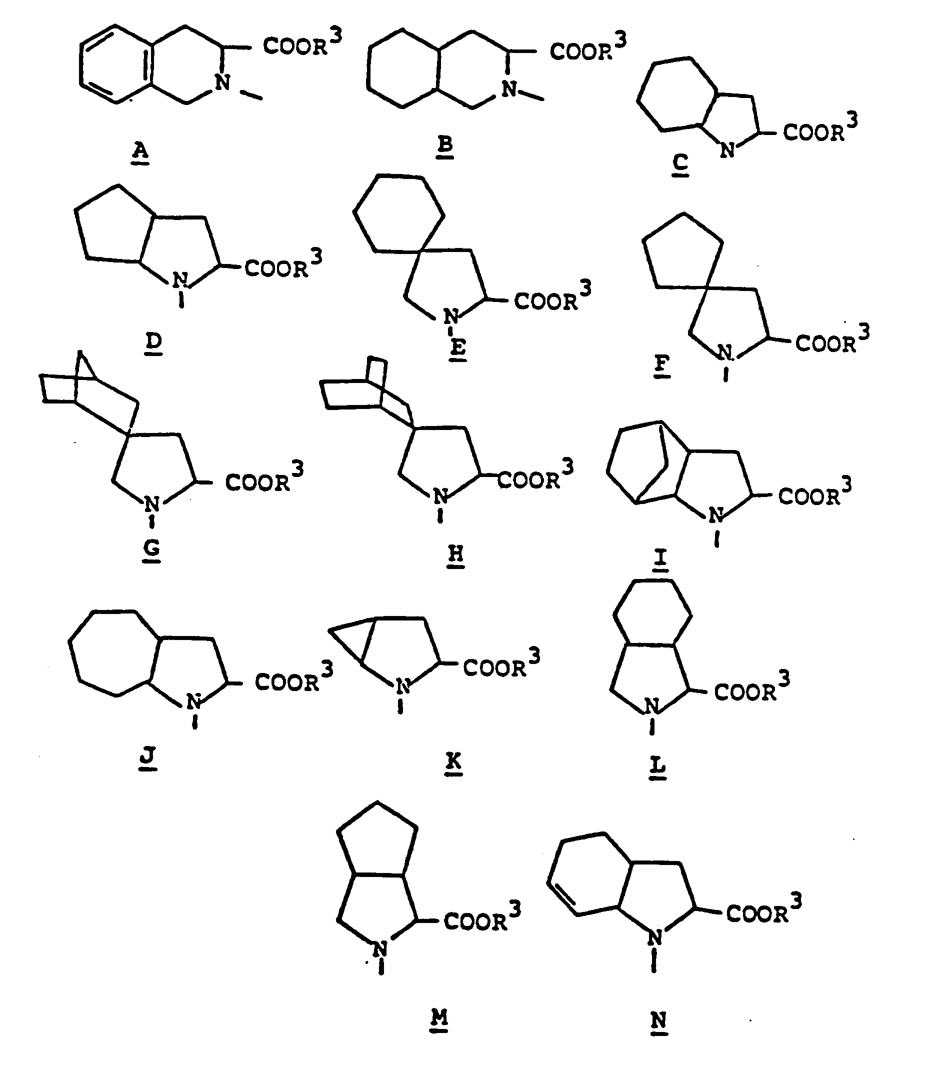

Insbesondere bevorzugt ist die Anwendung von Verbindungen der Formel I, in welcher n= 2 ist, R= Phenyl, R¹= Methyl, R² und R³ gleiche oder verschiedene (C₁-C₆)-Alkylreste oder (C₇-C₁₀)-Aralkylreste wie Benzyl oder Nitrobenzyl bedeuten und
R⁴ und R⁵ zusammen für einen Rest der Formel steht, 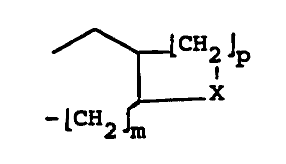
worin m= O oder 1, p= 0, 1 oder 2 und X= -CH₂-, -CH₂-CH₂- oder -CH=CH- bedeuten, wobei ein mit X gebildeter 6-Ring auch ein Benzolring sein kann.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Cninazolyl, Cinnolinyl, Pteridinyl, Oxyzolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls R¹ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen in der Peptidchemie üblichen Grupppen bevorzugt (vgl. Houben-Weyl, Bd. XV/1 und XV/2). Im Falle, daß R¹ die geschützte Lysin-Seitenkette bedeutet, werden die bekannten Amino-Schutzgruppen, insbesondere aber Z, Boc oder (C₁-₆)-Alkanoyl bevorzugt. Als O-Schutzgruppen für Tyrosin kommen bevorzugt (C₁-C₆)-Alkyl, insbesondere Methyl oder Ethyl in Frage.

ACE-Inhibitoren der Formel I lassen sich herstellen, indem man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiff'sche Basen reduziert und zum Schutz reaktiver Gruppen temporär eingeführte Schutzgruppen abspaltet und die erhaltenen Verbindungen gegebenenfalls in ihre physiologisch verträglichen Salze überführt.

In der genannten Weise kann man Verbindungen der Formel V mit Verbindungen der Formel VI umsetzen. 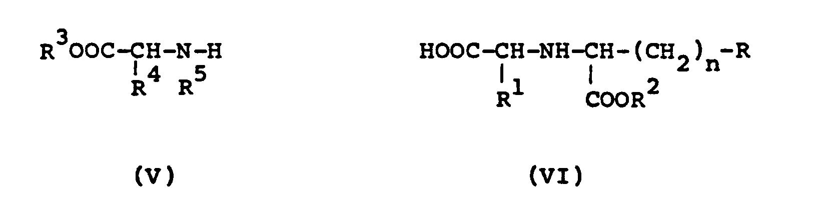
Die Umsetzung dieser Verbindungen kann beispielsweise in Analogie zu bekannten Peptidkupplungsverfahren in Gegenwart von Kupplungshilfsmitteln, wie Carbodiimiden (z.B. Dicyclohexylcarbodiimid), Diphenylphospharylazid, Alkanphosphorsäureanhydriden, Dialkylphosphinsäureanhydriden oder N,N-Succinylimidylcarbonate in CH₃CN. Aminogruppen in Verbindungen der Formel V können mit Tetraethyldiphosphit aktiviert werden. Die Verbindungen der Formel VI können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76-136).

Ebenso lassen sich auch Verbindungen der Formel VII mit Verbindungen der Formel VIII unter Bildung von Verbindungen der Formel I umsetzen, 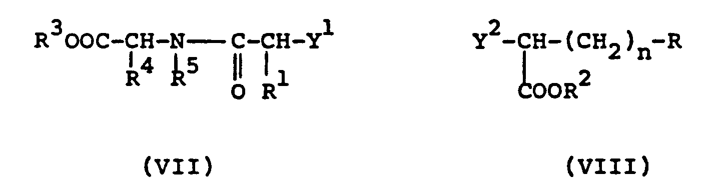
worin entweder Y¹ für Amino und Y² für eine Abgangsgruppe oder Y¹ für eine Abgangsgruppe und Y² für Amino stehen. Geeignete Abgangsgruppen sind z.B. Cl, Br, J, Alkylsulfonyloxy oder Arylsulfonyloxy.

Alkylierungen dieser Art führt man zweckmäßigerweise in Wasser oder einem organischen Lösungsmittel in Gegenwart einer Base durch.

Des weiteren lassen sich Verbindungen der Formel IX mit Verbindungen der Formel X kondensieren, 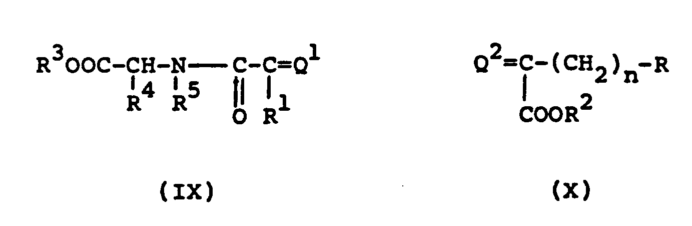
worin entweder Q¹ für Amino + Wasserstoff und Q² für Oxo steht oder Q¹ für Oxo und Q² für Amino + Wasserstoff steht.

Die Kondensation wird zweckmäßigerweise in Wasser oder einem organischen Lösungsmittel, wie einem niederen Alkohol, in Gegenwart eines Reduktionsmittels, wie NaBH₃CN, durchgeführt, wobei Verbindungen der Formel I direkt erhalten werden. Man kann aber auch die als Zwischenprodukte entstehenden Schiff'schen Basen oder Enamine gegebenenfalls nach vorheriger Isolierung unter Bildung von Verbindungen der Formel I reduzieren, beispielsweise durch Hydrierung in Gegenwart eines Übergangmetallkatalysators.

Schließlich führt auch die Umsetzung von Verbindungen der Formel IX (Q¹ = H + NH₂) mit Verbindungen der Formel XI oder deren Umsetzung mit Verbindungen der Formeln XII und XIII zu Verbindungen der Formel I (n=2),

R²OOC-CH=CH-CO-R (XI)

OCH-COOR² (XII)

R-CO-CH₃ (XIII)

wobei intermediär entstehende Schiff'sche Basen reduziert und eine Carbonylgruppe reduktiv in Methylen überführt werden.

In den oben genannten Formeln V - XIII sind R-R⁵ und n wie in Formel I definiert. Temporär zum Schutz nicht an der Reaktion beteiligter reaktiver Gruppen eingeführte Schutzgruppen werden nach beendeter Reaktion in an sich bekannter Weise abgespalten (vgl. Schröder, Lübke, loc cit., Seiten 1 - 75 und 246-270).

Besonders vorteilhaft können die folgenden Verbindungen nach der erfindungsgemäßen Methode angewendet werden:
N-(1-S-Carbethoxy-3-phenyl-propyl)-S-alanyl-S-1,2,3,4-tetrahydroisochinolin-3-carbonsäure
N-(1-S-Carbethoxy-3-cyclohexyl-propyl)-S-alanyl-S-1,2,3,4-tetrahydroisochinolin-3-carbonsäure
N-(1-S-Carbethoxy-3-phenyl-propyl)-S-lysyl-S-1,2,3,4-tetrahydroisochinolin-3-carbonsäure
N-(1-S-Carbethoxy-3-phenyl-propyl)-O-ethyl-S-tyrosyl-S-1,2,3,4-tetrahydroisochinolin-3-carbonsäure
N-(1-S-Carbethoxy-3-phenyl-propyl)-S-alanyl-3S-decahydroisochinolin-3-carbonsäure
N-(1-S-Carbethoxy-3-phenyl-propyl)-S-alanyl-(2S,3aS,7aS)-octahydroindol-2-carbonsäure
N-(1-S-Carbethoxy-3-cyclohexyl-propyl)-S-alanyl-(2S,3aS, 7aS)-octahydroindol-2-carbonsäure
N-(1-S-Carbethoxy-3-phenyl-propyl)-S-lysyl-(2S,3aS,7aS)-octahydroindol-2-carbonsäure
N-(1-S-Carbethoxy-3-cyclohexyl-propyl)-S-lysyl-(2S,3aS, 7aS)-octahydroindol-2-carbonsäure
N-(1-S-Carbethoxy-3-cyclohexyl-propyl)-S-lysyl-(2S,3aS, 7aS)-octahydroindol-2-carbonsäure
N-(1-S-Carbethoxy-3-phenyl-propyl)-O-methyl-S-tyrosyl-(2S, 3aS,7aS)-octahydroindol-2-carbonsäure
N-(1-S-Carbethoxy-3-phenyl-propyl)-O-ethyl-S-tyrosyl-(2S,3aS,7aS)-octahydroindol-2-carbonsäure
N-(1-S-Carbethoxy-3-(3,4-dimethylphenyl-propyl)-S-alanyl-(2S,3aS,7aS)-octahydroindol-2-carbonsäure
N-[1-S-Carbethoxy-3-(4-fluorphenyl)-propyl]-S-alanyl-(2S,3aS,7aS)-octahydroindol-2-carbonsäure
N-[1-S-Carbethoxy-3-(4-methoxyphenyl)-propyl]-S-alanyl-(2S,3aS,7aS)-octahydroindol-2-carbonsäure
N-[1-S-Carbethoxy-3-(3,4-dimethoxyphenyl)-propyl]-S-alanyl-(2S,3aS,7aS)-octahydroindol-2-carbonsäure
N-(1-S-Carbethoxy-3-cyclopentylpropyl)-S-alanyl-(2S,3aS, 7aS)-octahydroindol-2-carbonsäure
N-(1-S-Carbethoxy-3-phenyl-propyl)-S-alanyl-(2S,3aR,7aS)-octahydroindol-2-carbonsäure
N-(1-S-Carbethoxy-3-cyclohexyl-propyl)-S-alanyl-(2S,3aR,7aS)-octahydroindol-carbonsäure
N-(1-S-Carbethoxy-3-phenyl-propyl)-S-lysyl-(2S,3aR,7aS)-octahydroindol-2-carbonsäure
N-(1-S-Carbethoxy-3-cyclohexyl-propyl)-S-lysyl-(2S,3aR, 7aS)-octahydroindol-2-carbonsäure
N-(1-S-Carbethoxy-3-phenyl-propyl)-O-ethyl-S-tyrosyl-(2S,3aS,7aR)-octahydroindol-2-carbonsäure
N-(1-S-Carbethoxy-3-phenyl-propyl)-S-alanyl-(2S,3aR,7aR)-octahydroindol-2-carbonsäure
N-(1-S-Carbethoxy-3-phenyl-propyl)-S-lysyl-(2S,3aR,7aS)-octahydroindol-2-carbonsäure
N-(1-S-Carbethoxy-3-cyclohexyl-propyl)-S-alanyl-(2S,3aR, 7aR)-octahydroindol-2-carbonsäure
N-(1-S-Carbethoxy-3-cyclohexyl-propyl)-O-ethyl-S-tyrosyl-(2S,3aR,7aR)-octahydroindol-2-carbonsäure
N-(1-S-Carbethoxy-3-phenyl-propyl)-S-alanyl-(2S,3aS,7aR)-octahydroindol-2-carbonsäure
N-(1-S-Carbethoxy-3-phenyl-propyl)-O-ethyl-S-tyrosyl-(2S,3aS,7aS)-octahydroindol-2-carbonsäure
N-(1-S-Carbethoxy-3,4-dimethylphenyl-propyl)-S-alanyl-(2S,3aS,7aS)-octahydroindol-2-carbonsäure
N-[1-S-Carbethoxy-3-(4-fluorphenyl)-propyl]-S-alanyl-(2S,3aS,7aS)-octahydroindol-2-carbonsäure
N-[1-S-Carbethoxy-3-(4-methoxyphenyl)-propyl]-S-alanyl-(2S,3aS,7aS)-octahydroindol-2-carbonsäure
N-[1-S-Carbethoxy-3-(3,4-dimethoxyphenyl)-propyl]-S-alanyl-(2S,3aS,7aS)-octahydroindol-2-carbonsäure
N-(1-S-Carbethoxy-3-cyclopentylpropyl)-S-alanyl-(2S,3aS, 7aS)-octahydroindol-2-carbonsäure
N-(1-S-Carbethoxy-3-phenyl-propyl)-S-alanyl-cis-endo-2-azabicyclo[3.3.0]octan-3-S-carbonsäure
N-(1-S-Carbethoxy-3-phenyl-propyl)-S-lysyl-cis-endo-2-azabicyclo[3.3.0]octan-3-S-carbonsäure
N-(1-S-Carbethoxy-3-cyclohexyl-propyl)-S-alanyl-cis-endo-2-azabicyclo[3.3.0]octan-3-S-carbonsäure
N-(1-S-Carboxy-3-cyclohexyl-propyl)-S-alanyl-cis-endo-2-azabicyclo[3.3.0]octan-3-S-carbonsäure
N-(1-S-Carbethoxy-butyl)-S-alanyl-cis-endo-2-azabicyclo[3.3.0]octan-3-S-carbonsäure
N-(1-S-Carbethoxy-3-(3,4-dimethoxyphenylpropyl)-S-alanyl-cis-endo-2-azabicyclo[3.3.0]octan-3-S-carbonsäure
N-(1-S-Carbethoxy-3-cyclopentyl-propyl)-S-alanyl-cis-endo-azabicyclo-[3.3.0]octan-3-S-carbonsäure
N-(1-S-Carbethoxy-3-phenyl-propyl)-O-methyl-S-tyrosyl-cis-endo-2-azabicyclo[3.3.0]octan-3-S-carbonsäure
N-(1-S-Carbethoxy-3-phenyl-propyl)-O-ethyl-S-tyrosyl-cis-endo-2-azabicyclo[3.3.0]octan-3-S-carbonsäure
N-(1-S-Carbethoxy-3-(4-fluorphenyl-propyl)-S-alanyl-cis-endo-azabicyclo[3.3.0]octan-3-S-carbonsäure
N-(1-S-Carbethoxy-3-(4-methoxyphenyl-propyl)-S-alanyl-cis-endo-2-azabicyclo[3.3.0]octan-3-S-carbonsäure
N-(1-S-Carbethoxy-3-phenyl-propyl)-S-lysyl-(2S,3aR,6aS)-octahydrocyclopenta[b]pyrrol-2-carbonsäure
N-(1-S-Carbethoxy-3-cyclohexylpropyl)-lysyl-(2S,3a,6aS)-octahydrocyclopenta[b]pyrrol-2-carbonsäure
N-(1-S-Carbethoxy-3-phenyl-propyl)-O-ethyl-S-tyrosyl-(2S, 3aR,6aS)-octahydrocyclopenta[b]pyrrol-2-carbonsäure
N-(1-S-Carbethoxy-3-phenyl-propyl)-S-alanyl-2-(2S,3aR,6aS)-octahydrocyclopenta[b]pyrrol-2-carbonsäure
N-(1-S-Carbethoxy-3-phenyl-propyl)-S-alanyl-2-azaspiro-[4,5]decan-3-S-carbonsäure
N-(1-S-Carbethoxy-3-phenyl-propyl)-O-ethyl-2-tyrosyl-azaspiro-[4,5]decan-3-S-carbonsäure
N-(1-S-Carbethoxy-3-phenyl-propyl)-S-lysyl-2-azaspiro-[4,5]decan-3-S-carbonsäure
N-(1-S-Carbethoxy-3-cyclohexylpropyl)-S-alanyl-2-azaspiro [4,5]decan-3-S-carbonsäure
N-(1-S-Carbethoxy-3-cyclohexylpropyl)-S-lysyl-2-azaspiro-[4,5]decan-3-S-carbonsäure
N-(1-S-Carbethoxy-3-phenyl-propyl)-S-alanyl-2-azaspiro-[4,4]nonan-3-S-carbonsäure
N-(1-S-Carbethoxy-3-phenyl-propyl)-O-ethyl-S-tyrosyl-2-azaspiro[4,4]nonan-3-S-carbonsäure
N-(1-S-Carbethoxy-3-phenyl-propyl)-S-lysyl-2-azaspiro-[4,4]nonan-3-S-carbonsäure
N-(1-S-Carbethoxy-3-cyclohexyl-propyl)-S-alanyl-2-azaspiro[4,4]nonan-3-S-carbonsäure
N-(1-S-Carbethoxy-3-cyclopentyl-propyl)-S-alanyl-2-azaspiro[4,4]nonan-3-S-carbonsäure
N-(1-S-Carbethoxy-3-cyclopentyl-propyl)-S-lysyl-2-azaspiro[4,4]nonan-3-S-carbonsäure
N-(1-S-Carbethoxy-3-phenyl-propyl)-S-alanyl-spiro[bicyclo[2.2.1]heptan-2,3'-pyrrolidin]-5'-S-carbonsäure
N-(1-S-Carbethoxy-3-phenyl-propyl)-O-ethyl-S-tyrosylspiro[bicyclo[2.2.1]heptan-2,3'-pyrrolidin]-5'-S-carbonsäure
N-(1-S-Carbethoxy-3-phenyl-propyl)-S-lysyl-spiro[bicyclo[2.2.1]heptan-2,3'-pyrrolidin]-5'-S-carbonsäure
N-(1-S-Carbethoxy-3-cyclohexyl-propyl)-S-alanyl-spiro[bicyclo[2.2.1]heptan-2,3'-pyrrolidin]5'-S-carbonsäure
N-(1-S-Carbethoxy-3-cyclohexyl-propyl)-S-lysyl-spiro[bicyclo[2.2.1]heptan-2,3'-pyrrolidin]-5'-S-carbonsäure
N-(1-S-Carbethoxy-3-phenyl-propyl)-S-alanyl-spiro[bicyclo[2.2.2]octan-2,3'-pyrrolidin]-5'-S-carbonsäure
N-(1-S-Carbethoxy-3-phenyl-propyl)-O-ethyl-tyrosyl-spiro[bicyclo[2.2.2]octan-2,3'-pyrrolidin]-5'-S-carbonsäure
N-(1-S-Carbethoxy-3-phenyl-propyl)-S-lysyl-spiro[bicyclo[2.2.2]octan-2,3'-pyrrolidin]-5'-S-carbonsäure
N-(1-S-Carbethoxy-3-cyclohexyl-propyl)-S-alanyl-spiro[bicyclo[2.2.2]octan-2,3'-pyrrolidin]-5'-S-carbonsäure
N-(1-S-Carbethoxy-3-phenyl-propyl)-S-alanyl-2-azatricyclo[4,3,0,1^{6,9}]decan-3-S-carbonsäure
N-(1-S-Carbethoxy-3-phenyl-propyl)-O-ethyl-S-tyrosyl-2-azatricyclo[4,3,0,1^{6,9}]decan-3-S-carbonsäure
N-(1-S-Carbethoxy-3-phenyl-propyl)-S-lysyl-2-azatricyclo[4,3,0,1^{6,9}]decan-3-S-carbonsäure
N-(1-S-Carbethoxy-3-cyclohexyl-propyl)-S-alanyl-2-azatricyclo[4,3,0,1^{6,9}]decan-3-S-carbonsäure
N-(1-S-Carbethoxy-3-cyclohexyl-propyl)-S-lysyl-2-azatricyclo[4,3,0,1^{6,9}]decan-3-S-carbonsäure
N-(1-S-Carbethoxy-3-phenyl-propyl)-S-alanyl-decahydrocyclohepta[b]pyrrol-2-S-carbonsäure
N-(1-S-Carbethoxy-3-phenyl-propyl)-O-ethyl-S-tyrosyldecahydrocyclophepta[b]pyrrol-2-S-carbonsäure
N-(1-S-Carbethoxy-3-phenyl-propyl)-S-lysyl-decahydrocyclohepta[b]pyrrol-2-S-carbonsäure
N-(1-S-Carbethoxy-3-cyclohexyl-propyl)-S-alanyl-decahydrocyclohepta[b]pyrrol-2-S-carbonsäure
N-(1-S-Carbethoxy-3-cyclohexyl-propyl)-S-lysyl-decahydrocyclohepta[b]pyrrol-2-S-carbonsäure
N-(1-S-Carbethoxy-3-phenyl-propyl)-S-alanyl-trans-octahydroisoindol-1-S-carbonsäure
N-(1-S-Carbethoxy-3-phenyl-propyl)-S-alanyl-cis-octahydroisoindol-1-S-carbonsäure
N-(1-S-Carbethoxy-3-cyclohexyl-propyl)-S-alanyl-transoctahydroisoindol-1-S-carbonsäure
N-(1-S-Carbethoxy-3-cyclohexyl-propyl)-S-alanyl-cis-octahydroisoindol-1-S-carbonsäure
N-(1-S-Carbethoxy-3-phenyl-propyl)-S-alanyl-cis-octahydrocyclopenta[c]pyrrol-1-S-carbonsäure
N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-cyclohexyl-propyl)-S-lysyl-cis-octahydrocyclopenta[c]pyrrol-1-S-carbonsäure
N-(1-S-Carbethoxy-3-phenyl-propyl)-S-alanyl-2,3,3a,4,5,7a-hexahydroindol-cis-endo-2-S-carbonsäure
N-(1-S-Carbethoxy-3-phenyl-propyl)-S-lysyl-2,3,3a,4,5,7a-hexahydroindol-cis-endo-2-S-carbonsäure
N-(1-S-Carbethoxy-3-cyclohexyl-propyl)-S-lysyl-2-azabicyclo[3.1.0]hexan-3-S-carbonsäure
N-(1-S-Carboxy-3-phenyl-propyl)-S-lysyl-2-azabicyclo[3.1.0]hexan-cis-endo-3-S-carbonsäure
N-(1-S-Carbethoxy-3-cyclopentylpropyl)-S-alanyl-2-azabicylco[3.1.0]hexan-3-carbonsäure
N-(1-S-Carbethoxy-3-phenyl-propyl)-S-alanyl-cis-endo-2-azabicyclo[3.1.0]hexan-3-S-carbonsäure
N-(1-S-Carbethoxy-3-cyclohexyl-propyl)-S-alanyl-cis-endo-2-azabicyclo[3.1.0]hexan-3-S-carbonsäure
Diese Verbindungen lassen sich z.B. nach dem in der deutschen Patentanmeldung P 33 33 455.2 beschriebenen Verfahren herstellen, in dem die in der Anmeldung beschriebenen tert. Butyl- oder Benzylreste in bekannter Weise durch saure oder alkalische Hydrolyse oder durch Edelmetall-katalysierte Hydrogenolyse in die Monocarbonsäurederivate überführt werden. Die N^{ε}-Benzyloxycarbonylschutzgruppe der Lysinderivate wird durch Edelmetallkatalysierte Hydrogenolyse entfernt. Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die Verbindungen der Formel I sind Hemmstoffe des Angiotensin-Converting-Enzymes (ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel I sind z.B. bekannt aus US-Patent 4 129 571, US-Patent 4 374 829, EP-A-79522, EP-A-79022, EP-A-49658, EP-A-51301, US-Patent 4 454 292, US-Patent 4 374 847, EP-A-72352, US-Patent 4 350 704, EP-A-50800, EP-A-46953, US-Patent 4 344 949, EP-A-84164, US-Patent 4 470 972, EP-A-65301 und EP-A-52991.

Vorteilhaft sind auch oral wirksame ACE-Inhibitoren, wie z.B. Ramipril, Enalapril, Captopril, Lisinopril, Perindopril, Cilazapril, RHC 3659, CGS 13945, CGS 13928C, CGS 14824A, CI-906, SCH 31846, Zofenopril, Fosenopril, Alacepril und andere. Oral wirksame ACE-Inhibitoren sind beispielsweise in Brunner et al., J. Cardiovasc. Pharmacol. 7 (Suppl. I) [1985] S2-S11 beschrieben.

Bevorzugt sind die aus der EP-A-79022 bekannten ACE-Inhibitoren der Formel III 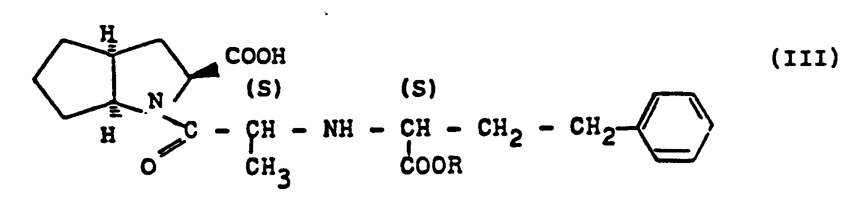
in welcher
R Wasserstoff, Methyl, Ethyl oder Benzyl
bedeutet,
insbesondere die Verbindung der Formel III, worin R=Ethyl bedeutet (Ramipril).

Weiterhin bevorzugt sind die aus der EP-A-84164 bekannten ACE-Inhibitoren der Formel IV 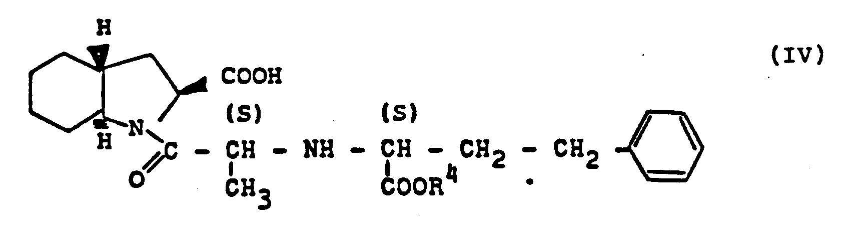
in welcher
R⁴ Wasserstoff, (C₁-C₄)-Alkyl oder Benzyl bedeutet, insbesondere die Verbindung der Formel IV,
worin R⁴=Ethyl bedeutet.

In Ausübung der erfindungsgemäßen Methode können die oben beschriebenen Angiotensin-Converting-Enzyme-Inhibitoren an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen, wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können verträgliche, organische bzw. anorganische Begleitstoffe, beispielsweise Granulierstoffe, Klebe- und Bindemittel, Gleitmittel, Suspendiermittel, Lösungsmittel, antibakterielle Mittel, Netzmittel und Konservierungsmittel enthalten. Orale und parenterale Anwendungsformen werden bevorzugt. Die Verbindungen der Formel I können in Dosierungen von 0,1 - 50 mg pro Dosis ein- bis dreimal täglich verabreicht werden.

Die ACE-Inhibitoren können erfindungsgemäß auch kombiniert mit den Prostaglandin-Stoffwechsel beeinflussenden Stoffen angewendet werden. Solche Stoffe sind beispielsweise stabile Prostacyclinanaloge, Thromboxan-Synthetase-Hemmer und Thromboxan-Antagonisten.

Die Erfindung betrifft daher auch pharmazeutische Zubereitungen, enthaltend a) einen ACE-Inhibitor oder dessen physiologisch verträgliches Salz und b) einen den Prostaglandin-Stoffwechsel beeinflussenden Stoff oder dessen physiologisch verträgliches Salz und ihre Anwendung bei der Behandlung der Atherosklerose, der Thrombose und/oder der peripheren Gefäßkrankheit.

Die Erfindung betrifft weiterhin ganz allgemein Erzeugnisse, enthaltend die vorstehend unter a) und b) genannten Stoffe als Kombinationspräparat zur gleichzeitigen, getrennten oder zeitlich abgestuften Anwendung bei der Behandlung der Atherosklerose, der Thrombose und/oder der peripheren Gefäßkrankheit.

In der Entwicklung der Atherosklerose spielt eine erhöhte Aggregierbarkeit der Blutplättchen eine besonders wichtige Rolle. Folgeerkrankungen sind beispielsweise Thrombosen und periphere Gefäßkrankheit; diese Krankheiten sind die Hauptursache für die erhöhte Morbidität und Mortalität bei erhöhtem Blutdruck. Blutplättchen enthalten ein Angiotensin-I-prozessierendes System und tragen auf ihrer Membran hochaffine Bindungsstellen für Angiotensin II. Die überwiegende Lokalisierung des Angiotensin-Converting-Enzyme (ACE) auf der luminalen Plasma-Membran der Endothelzellen legen Plättchen-Endothel-Interaktionen bei der lokalen Angiotensin II-Produktion nahe; ACE-Hemmer können hierbei interferieren. Weiterhin potenziert die Inhibierung des ACE die Wirkung des Bradykinin, indem dessen Abbau verhindert wird. Von Bradykinin ist bekannt, daß es ein starker Stimulator der Prostacyclin-Freisetzung aus Endothelzellen ist; Bradykinin wiederum ist ein potenter Hemmer der Plättchenaggregation.

Die Wirksamkeit der Verbindungen der Formel I auf die Plättchenaggregation und damit auf Atherosklerose, Thrombose und periphere Gefäßkraflkheit sowie andere Krankheitszustände, bei denen eine erhöhte Aggregabilität der Blutplättchen vorliegt, kann aus verschiedenen Testmodellen abgeleitet werden.

Als Beispiele sollen im folgenden jeweils die Ergebnisse mit N-(1-S-Carbethoxy-3-phenyl-propyl)-S-alanyl-cis-endo-2-azabicyclo[3.3.0]octan-3-S-carbonsäure (Formel II) dienen.

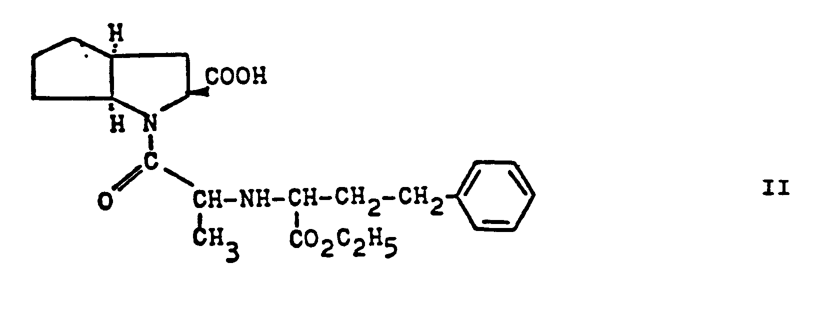

### A Ergebnisse in vitro

Plättchenreiches Kaninchenplasma wird wie von Born (Arzneimittel-Forsch. 31, 2012 (1981)) angegeben, gewonnen. Die Plättchenaggregation wird durch Erhöhung des Lichtdurchganges durch eine Küvette, die dieses Plasma enthält, gemessen. Die Plättchenzahl wird durch Verdünnung mit autologem, plättchenarmem Plasma auf 450000/mm³ eingestellt. Die Verbindung der Formel IIin Konzentrationen von 0.1-10 µg/ml Plasma hat keinen Einfluß auf die durch 0.24 mmol/l Arachidonsäure, 5 mmol/l ADP oder 4 µg/ml Kollagen ausgelöste Aggregation. Dagegen wird eine durch 4 µg/ml PGI₂ bewirkte Aggregationshemmung gegen Arachidonsäure durch die Verbindung der Formel II in genannten Dosisbereich auf 100% gesteigert.

### B Ergebnisse in vivo

1. Akute Studie
   Wache Kaninchen erhielten eine einmalige orale Dosis von 1,0 - 10,0 mg/kg der Verbindung der Formel II. Nach 1 Stunde werden die Tiere getötet und plättchenreiches Plasma gewonnen. Die Plättchenaggregation wird wie unter A) beschrieben bestimmt.
   Die Aggregation wird gegenüber den 3 dort beschriebenen Stimulatoren, insbesondere gegenüber Arachidonsäure, vermindert gefunden. Eine Potenzierung der PGI₂-Wirkung wird ebenfalls beobachtet.
2. Chronische Studie
   Wache Kaninchen erhielten 1 mg/kg/d der Verbindung der Formel II für 14 Tage, dann wurde wie unter 1) beschrieben weiter verfahren. In allen Tieren wird eine ausgeprägte Inhibierung der durch Arachidonsäure und ADP ausgelösten Plättchenaggregation gefunden.

Die folgenden Beispiele geben die Anwendungsformen zur Behandlung der Atherosklerose, der Thrombose und der peripheren Gefäßkrankheit nach der erfindungsgemäßen Methode an. Die Verbindungen der Formel I können analog den Beispielen in die entsprechenden Anwendungsformen gebracht werden.

### Beispiel 1

Herstellung des erfindungsgemäß verwendeten Mittels zur oralen Anwendung in der Behandlung der Atherosklerose, der Thrombose und der peripheren Gefäßkrankheit.

1000 Tabletten, die je 10 mg 1-N-(1-S-Carbethoxy-3-phenyl-propyl)-S-alanyl-1S,3S,5S-2-azabicyclo[3.3.0]octan-3-carbonsäure enthalten, werden mit den folgenden Hilfsmitteln hergestellt:

| | |
|---|---|
| N-(1-S-Carbethoxy-3-phenyl-propyl)-S-alanyl-1S,3S,5S-2-azabicyclo[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

N-(1-S-Carbethoxy-3-phenyl-propyl)-S-alanyl-1S,3S-5S,-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.
Diese Tabletten können zur Behandlung der Atherosklerose, der Thrombose und/oder der peripheren Gefäßkrankheit verwendet werden.

### Beispiel 2

Analog Beispiel 1 werden 1000 Tabletten hergestellt, die je 10 mg N-(1-S-Carbethoxy-3-phenyl-propyl)-S-alanyl-(2S,3aR,7aS)-octahydroindol-2-carbonsäure-hydrochlorid enthalten.

### Beispiel 3

Gelatine-Kapseln, die je 10 mg N-(1-S-Carbethoxy-3-phenyl-propyl)-S-alanyl-1S,3S,5S-2-azabicyclo[3.3.0]octan-3-carbonsäure enthalten, werden mit der folgenden Mischung gefüllt:

| | |
|---|---|
| N-(1-S-Carbethoxy-3-phenyl-propyl)-S-alanyl-1S,3S,5S-2-azabicyclo[3.3.0]octan-3-carbonsäure | 10 mg |
| Magnesiumstearat | 1 mg |
| Lactose | 214 mg |

Diese Kapseln können zur Behandlung der Atherosklerose, der Thrombose und/oder der peripheren Gefäßkrankheit verwendet werden.

### Beispiel 4

Die Herstellung einer Injektionslösung zur Behandlung der Atherosklerose, der Thrombose und/oder der peripheren Gefäßkrankheit wird im folgenden beschrieben:

| | |
|---|---|
| N-(1-S-Carboxy-3-phenyl-propyl)-S-alanyl-1S,3S,5S-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

N-(1-S-Carboxy-3-phenyl-propyl)-S-alanyl-1S,3S,5S-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

### Beispiel 5

Tabletten, die zur Behandlung der Atherosklerose, der Thrombose und/oder der peripheren Gefäßkrankheit verwendet werden können, werden wie in Beispiel 1 beschrieben hergestellt, mit der Ausnahme, daß anstelle von N-(1-S-Carbethoxy-3-phenyl-propyl)-S-alanyl-1S,3S,5S-2-azabicyclo[3.3.0]octan-3S-carbonsäure
N-(1-S-Carboxy-3-phenyl-propyl)-S-alanyl-1S,3S,5S-2-azabicyclo[3.3.0]octan-3-carbonsäure
oder N-(1-S-Carboxy-3-phenyl-propyl)-S-alanyl-2S,3aR,7aS-octahydroindol-2-carbonsäure oder
N-(1-S-Carbethoxy-3phenyl-propyl)-S-alanyl-cis-2,3,3a,-4,5,7a-hexahydro[1H]indol-2-S-endo-carbonsäure oder
N-(1-S-Carboxy-3-phenyl-propyl)-S-alanyl-cis-2,3,4a,4, 5,7a-hexahydro[1H]indol-2S-endo-carbonsäure oder
N-(1-S-Carboxy-3-phenyl-propyl)-S-lysyl-1S,3S,5S-2-azabicyclo[3.3.0]octan-3-carbonsäure oder
N-(1-S-Carbethoxy-3-cyclohexyl-propyl)-S-alanyl-1S,3S,5S-2-azabicyclo[3.3.0]octan-3-carbonsäure oder
N-(1-S-Carboxy-3-cyclohexyl-propyl)-S-lysyl-1S-3S,5S-2-azabicylco[3.3.0]octan-3-carbonsäure angewendet werden.

### Beispiel 6

Eine Injektionslösung wird analog der in Beispiel 4 beschriebenen Vorschrift hergestellt, mit der Ausnahme, daß anstelle von N-(1-S-Carbethoxy-3-phenyl-propyl)-S-alanyl-1S,3S,5S-2-azabicyclo[3.3.0]octan-3-carbonsäure
N-(1-S-Carboxy-3-phenyl-propyl)-S-alanyl-1S,3S,5S-2-azabicyclo[3.3.0]octan-3-carbonsäure oder
N-(1-S-Carbethoxy-3-phenyl-propyl)-S-alanyl-2S,3aR,7aS-octahydroindol-2-carbonsäurehydrochlorid oder
N-(1-S-Carboxy-3-phenyl-propyl)-S-alanyl-2S,3aR,7aS-octahydroindol-2-carbonsäure oder
N-(1-S-Carbethoxy-3-cyclohexyl-propyl)-S-alanyl-cis-2,3,3a,4,5,7a-hexahydro[1H]indol-2-S-endo-carbonsäure oder
N-(1-S-Carboxy-3-phenyl-propyl)-S-alanyl-cis-2,3,3a,4,5,7a-hexahydro[1H]indol-2-S-endo-carbonsäure oder
N-(1-Carboxy-3-phenyl-propyl)-S-lysyl-1S,3S,5S-2-azabicyclo[3.3.0]octan-3-carbonsäure oder
N-(1-S-Carbethoxy-3-cyclohexyl)-S-alanyl-1S,3S,5S-2-azabicyclo[3.3.0]octan-3-carbonsäure oder
N-(1-S-Carboxy-3-cyclohexyl-propyl)-S-lysyl-1S,3S,5S-2-azabicyclo[3.3.0]octan-3-carbonsäure angewendet werden.

## Patentansprüche

1. Verwendung eines Angiotensin-Converting-Enzyme-Inhibitors oder dessen physiologisch verträglichen Salze bei der Herstellung eines pharmazeutischen Mittels zur Behandlung der Atherosklerose, der Thrombose und/oder der peripheren Gefäßkrankhert in Säugern, dadurch gekennzeichnet, daß ein Angiotensin-Converting-Enzyme-Inhibitor der Formel I eingesetzt wird 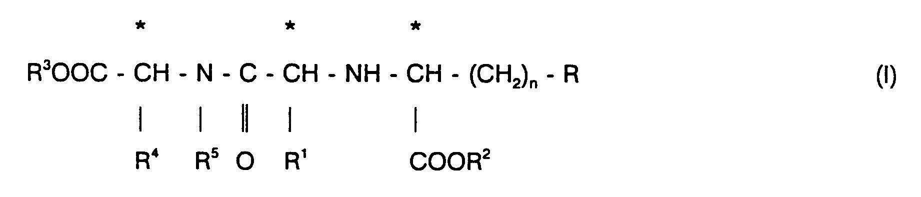 in welcher
n = 1 oder 2 ist,
R = Aryl mit 6 - 12 C-Atomen, das durch (C₁-C₄)--Alkyl, (C₁-C₄)-Alkoxy, Hydroxy, Halogen, Nitro, Amino, Aminomethyl, (C₁-C₄)-Alkylamino, Di-(C₁-C₄)-alkylamino, (C₁-C₄)-Alkanoylamino, Methylendioxy, Carboxy, Cyano und/oder Sulfamoyl mono-, di- oder trisubstituiert sein kann,
R¹ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
(C₃-C₉)-Cycloalkyl-(C₁-C₄)-alkyl,
(C₅-C₉)-Cycloalkenyl-(C₁-C₄)-alkyl,
gegebenenfalls teilhydriertes Aryl mit 6 - 12 C-Atomen, das wie oben bei R beschrieben substituiert sein kann,
(C₆-C₁₂)-Aryl-(C₁-C₄)-alkyl oder (C₇-C₁₃)-Aroyl-(C₁ oder C₂)alkyl die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, die gegebenenfalls geschützte Seitenkette natürlich vorkommenden α-Aminosäure R¹-CH(NH₂)-COOH bedeuten,
R² und R³ gleich oder verschieden sind und Wasserstoff,
Alkyl mit 1 - 6 C-Atomen,
Alkenyl mit 2 - 6 C-Atomen,
Di-(C₁-C₄)-alkylamino-(C₁-C₄)-alkyl,
(C₁-C₅)-Alkanoyloxy-(C₁-C₄)-alkyl,
(C₁-C₆)-Alkoxy-carbonyloxy-(C₁-C₄)-alkyl,
(C₇-C₁₃)-Aroyloxy-(C₁-C₄)-alkyl,
(C₆-C₁₂)-Aryloxycarbonyloxy-(C₁-C₄)-alkyl,
Aryl mit 6 - 12 C-Atomen,
(C₆-C₁₂)-Aryl-(C₁-C₄)-alkyl,
(C₃-C₉)-Cycloalkyl oder
(C₃-C₉)-Cycloalkyl-(C₁-C₄)-alkyl
bedeuten und
worin R⁴ und R⁵ zusammen mit den sie tragenden Atomen für ein System aus der Reihe Tetrahydroisochinolin, Decahydroisochinolin, Octahydroindol, Octahydrocyclopenta[b]pyrrol, 2-Aza-spiro[4.5]decan, 2-Azaspiro[4.4]nonan, Spiro[(bicyclo-[2.2.1]heptan)-2,3'-pyrrolidin], Spiro[(bicyclo-[2.2.2]octan)-2,3'-pyrrolidin], 2-Azatricyclo[4,3,0,1^{6,9}]decan, Decahydrocyclopenta[b]pyrrol, Octahydroisoindol, Octahydrocyclopenta[c]pyrrol, 2,3,3a,4,5,7a-Hexahydroindol und 2-Azabicyclo[3.1.0]hexan stehen.

2. Verwendung gemäß Anspruch 1, dadurch gekennzeichnet, daß [S,S,S,S,S]-N-(1-Carbethoxy-3-phenyl-propyl)-alanyl-octahydroindol-2-carbonsäure, N-[1-(S)-Carbethoxy-3-phenyl-propyl)-(S)-alanyl]-2S, 3aR, 7aS-octahydroindol-2-carbonsäure, [S,S,S,S,S]-N-[(1-Carbethoxy-3-phenyl-propyl)-alanyl]-decahydroisochinolin-3-carbonsäure, [S,S,S]-N-[(1-Carbethoxy-3-phenyl-propyl)-alanyl]-tetrahydroisochinolin-3-carbonsäure, N-(1-S-Carbethoxy-3-phenyl-propyl)-S-alanyl-cis-end-2-azabicyclo[3.1.0]hexan-3-S-carbonsäure oder N-(1-S-Carbethoxy-3-phenyl-propyl)-S-alanyl-cis-endo-2,3,3a,4,5,7a-hexahydroindol-2-S-carbonsäure angewendet wird.

3. Verwendung gemäß Anspruch 1, dadurch gekennzeichnet, daß (S,S,S,S,S)-N-(1-Carbethoxy-3-phenyl-propyl)-alanyl-2-azabicyclo[3,3,0]octan-3-carbonsäure angewendet wird.

4. Verwendung gemäß Anspruch 3, dadurch gekennzeichnet, daß anstelle der Ethylester die entsprechenden Dicarbonsäuren angewendet werden.

## Claims

1. The use of an angiotensin converting enzyme inhibitor or its physiologically tolerated salts in the preparation of a pharmaceutical composition for the treatment of atherosclerosis, of thrombosis and/or of peripheral vessel disease in mammals, which comprises employing an angiotensin converting enzyme inhibitor of the formula I 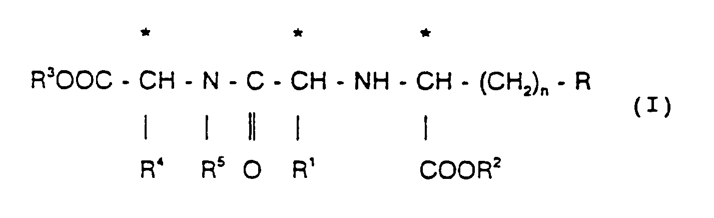 in which
n is 1 or 2,
R denotes aryl which has 6-12 carbon atoms and can be mono-, di- or trisubstituted by (C₁-C₄)-alkyl, (C₁-C₄)-alkoxy, hydroryl, halogen, nitro, amino, aminomethyl, (C₁-C₄)-alkylamino, di-(C₁-C₄)-alkylamino, (C₁-C₄)-alkanoylamino, methylenedioxy, carboxyl, cyano and/or sulfamoyl,
R¹ denotes hydrogen, alkyl having 1-6 carbon atoms, alkenyl having 2-6 carbon atoms, alkynyl having 2-6 carbon atoms, cycloalkyl having 3-9 carbon atoms, cycloalkenyl having 5-9 carbon atoms, (C₃-C₉)-cycloalkyl-(C₁-C₄)-alkyl, (C₅-C₉)-cycloalkenyl-(C₁-C₄)-alkyl, optionally partially hydrogenated aryl which has 6-12 carbon atoms and can be substituted as described above for R, (C₆-C₁₂)-aryl-(C₁-C₄)-alkyl or (C₇-C₁₃)-aroyl-(C₁ or C₂)-alkyl, both of which can be substituted as the abovementioned aryl, mono- or bicyclic, optionally partially hydrogenated heteroaryl which has 5-7 or 8-10 ring atoms respectively, 1 to 2 of these ring atoms representing sulfur or oxygen atoms and/or 1 to 4 of these ring atoms representing nitrogen atoms, or the optionally protected side chain of a naturally occurring α-amino acid R¹-CH(NH₂)-COOH,
R² and R³ are identical or different and denote hydrogen, alkyl having 1-6 carbon atoms, alkenyl having 2-6 carbon atoms, di-(C₁-C₄)-alkylamino-(C₁-C₄)-alkyl, (C₁-C₅)-alkanoyloxy-(C₁-C₄)-alkyl, (C₁-C₆)-alkoxycarbonyloxy-(C₁-C₄)-alkyl, (C₇-C₁₃)-aroyloxy-(C₁-C₄)-alkyl, (C₆-C₁₂)-aryloxycarbonyloxy-(C₁-C₄)alkyl, aryl having 6-12 carbon atoms, (C₆-C₁₂)-aryl-(C₁-C₄)-alkyl, (C₃-C₉)-cycloalkyl or (C₃-C₉)-cycloalkyl-(C₁-C₄)-alkyl, and
in which R⁴ and R⁵ together with the atoms carrying them represent a system from the series comprising tetrahydroisoquinoline, decahydroisoquinoline, octahydroindole, octahydrocyclopenta[b]pyrrole, 2-azaspiro[4.5]decane, 2-azaspiro[4.4]nonane, spiro[(bicyclo[2.2.1]heptane)-2,3'-pyrrolidine], spiro[(bicyclo[2.2.2]octane)-2,3'-pyrrolidine], 2-azatricyclo[4.3.0.1^{6,9}]decane, decahydrocyclopenta[b]pyrrole, octahydroisoindole, octahydrocyclopenta[c]pyrrole, 2,3,3a,4,5,7a-hexahydroindole and 2-azabicyclo[3.1.0]hexane.

2. The use as claimed in claim 1 wherein there is administration of [S,S,S,S,S]-N-(1-carboethoxy-3-phenylpropyl)-alanyl-octahydroindole-2-carboxylic acid, N-[1-(S)-carboethoxy-3-phenylpropyl)-(S)-alanyl]-2S,3aR,7aS-octahydroindole-2-carboxylic acid, [S,S,S,S,S]-N-[(1-carboethoxy-3-phenylpropyl)-alanyl]-decahydroisoquinoline-3-carboxylic acid, [S,S,S]-N-[(1-carboethoxy-3-phenylpropyl)-alanyl]-tetrahydroisoquinoline-3-carboxylic acid, N-(1-S-carboethoxy-3-phenylpropyl)-S-alanyl-cis-endo-2-azabicyclo[3.1.0]hexane-3-S-carboxylic acid or N-(1-S-carboethoxy-3-phenylpropyl)-S-alanyl-cis-endo-2,3,3a,4,5,7a-hexahydroindole-2-S-carboxylic acid.

3. The use as claimed in claim 1, wherein there is administration of (S,S,S,S,S)-N-(1-carboethoxy-3-phenylpropyl)-alanyl-2-azabicyclo[3.3.0]-octane-3-carboxylic acid.

4. The method as claimed in claim 3, wherein in place of the ethyl esters there is administration of the corresponding dicarboxylic acids.

## Revendications

1. Utilisation d'un inhibiteur de l'enzyme de conversion de l'angiotensine, ou de ses sels physiologiquement acceptables, dans la préparation d'un produit pharmaceutique destiné au traitement de l'athérosclérose, de la thrombose et/ou de l'angiopathie périphérique chez des mammifères, caractérisée en ce que l'on utilise un inhibiteur de l'enzyme de conversion de l'angiotensine de formule I 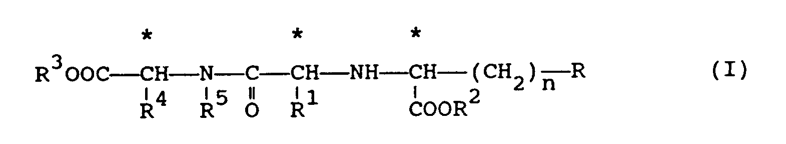 dans laquelle
n est 1 ou 2,
R représente un radical aryle ayant de 6 à 12 atomes de carbone, qui peut être mono-, di- ou trisubstitué par un ou des atomes d'halogène ou groupes alkyle en C₁-C₄, alcoxy en C₁-C₄, hydroxy, nitro, amino, aminométhyle, alkyl(C₁-C₄)-amino, dialkyl(C₁-C₄)amino, alcanoyl(C₁-C₄)-amino, méthylènedioxy, carboxy, cyano et/ou sulfamoyle,
R¹ représente un atome d'hydrogène, un radical alkyle ayant de 1 à 6 atomes de carbone, alcényle ayant de 2 à 6 atomes de carbone, alcynyle ayant de 2 à 6 atomes de carbone, cycloalkyle ayant de 3 à 9 atomes de carbone, cycloalcényle ayant de 5 à 9 atomes de carbone, cycloalkyl(C₃-C₉)-alkyle(C₁-C₄), cycloalcényl(C₅-C₉)-alkyle(C₁-C₄), un radical aryle ayant de 6 à 12 atomes de carbone, éventuellement partiellement hydrogéné, qui peut être substitué comme décrit plus haut à propos de R, un radical aryl(C₆-C₁₂)-alkyle(C₁-C₄) ou aroyl(C₇-C₁₃)-alkyle(C₁ ou C₂) qui peuvent l'un et l'autre être substitués comme le radical aryle précédent, un radical hétéroaryle mono- ou bicyclique, éventuellement partiellement hydrogéné, ayant respectivement 5-7 ou 8-10 atomes formant le ou les cycles, dont 1 ou 2 atomes formant le cycle représentent des atomes de soufre ou d'oxygène et/ou 1 à 4 atomes formant le cycle représentent des atomes d'azote, qui peut être substitué comme le radical aryle précédent, ou la chaîne latérale éventuellement protégée d'un α-aminoacide R¹-CH(NH₂)-COOH existant dans la nature,
R² et R³ sont identiques ou différents et représentent un atome d'hydrogène,
un radical alkyle ayant de 1 à 6 atomes de carbone,
alcényle ayant de 2 à 6 atomes de carbone,
dialkyl(C₁-C₄)amino-alkyle(C₁-C₄),
alcanoyloxy(C₁-C₅)-alkyle(C₁-C₄),
alcoxy(C₁-C₆)-carbonyloxy-alkyle(C₁-C₄),
aroyloxy(C₇-C₁₃)-alkyle(C₁-C₄),
aryloxy(C₆-C₁₂)-carbonyloxy-alkyle(C₁-C₄),
aryle ayant de 6 à 12 atomes de carbone,
aryl(C₆-C₁₂)-alkyle(C₁-C₄),
cycloalkyle en C₃-C₉ ou
cycloalkyl(C₃-C₉)-alkyle(C₁-C₄),
et dans laquelle
R⁴ et R⁵ représentent, conjointement avec les atomes qui les portent, un système choisi parmi les suivants: tétrahydroisoquinoléine, décahydroisoquinoléine, octahydroindole, octahydrocyclopenta[b]pyrrole, 2-azaspiro[4.4]nonane, spiro[(bicyclo[2.2.1]heptane)-2,3'-pyrrolidine], spiro[(bicyclo[2.2.2]octane)-2,3'-pyrrolidine], 2-azatricyclo[4.3.0.1^{6,9}]décane, décahydrocyclopenta[b]pyrrole, octahydroisoindole, octahydrocyclopenta[c]pyrrole, 2,3,3a,4,5,7a-hexahydroindole et 2-azabicyclo[3.1.0]hexane.

2. Utilisation selon la revendication 1, caractérisée en ce que l'on utilise l'acide [S,S,S,S,S]-N-(1-carbéthoxy-3-phénylpropyl)-alanyloctahydroindol-2-carboxylique, l'acide N-[1-(S)-carbéthoxy-3-phénylpropyl)-(S)-alanyl]-2S,3aR,7aS-octahydroindol-2-carboxylique, l'acide [S,S,S,S,S]-N-[(1-carbéthoxy-3-phénylpropyl)-alanyl]-décahydroisoquinoléine-3-carboxylique, l'acide [S,S,S]-N-[(1-carbéthoxy-3-phénylpropyl)-alanyl]-tétrahydroisoquinoléine-3-carboxylique, l'acide N-(1-S-carbéthoxy-3-phénylpropyl)-S-alanyl-*cis*-endo-2-azabicyclo[3.1.0]hexane-3-S-carboxylique ou l'acide N-(1-S-carbéthoxy-3-phénylpropyl)-S-alanyl-*cis*-endo-2,3,3a,4,5,7a-hexahydroindol-2-S-carboxylique.

3. Utilisation selon la revendication 1, caractérisée en ce que l'on utilise l'acide (S,S,S,S,S)-N-(1-carbéthoxy-3-phénylpropyl)-alanyl-2-azabicyclo[3.3.0)octane-3-carboxylique.

4. Utilisation selon la revendication 3, caractérisée en ce que, au lieu des esters éthyliques, on utilise les acides dicarboxyliques correspondants.
